# FASCICULE DE BREVET EUROPEEN

(11) **EP 1 061 929 B1**
(45) Date de publication et mention de la délivrance du brevet: **27.04.2005**
(21) Numéro de dépôt: 99939161.8
(22) Date de dépôt: 08.03.1999
(51) Int. Cl.: A61K 31/57, A61K 9/20

(54) **COMPOSITION PHARMACEUTIQUE A BASE DE PROGESTERONE NATURELLE DE SYNTHESE ET D'OESTRADIOL ET SON PROCEDE DE PREPARATION**
PHARMAZEUTISCHE ZUSAMMENSETZUNGEN ENTHALTEND SYNTETHISCH NATÜRLICHE PROGESTERON UND ESTRADIOL UND VERFAHREN ZUR HERSTELLUNG DERSELBEN
PHARMACEUTICAL COMPOSITION BASED ON NATURAL SYNTHESIS PROGESTERONE AND OESTRADIOL AND PREPARATION METHOD

(30) Priorité: 09.03.1998 FR 9802830
(43) Date de publication de la demande: 27.12.2000
(73) Titulaire: Laboratoires Besins Iscovesco, 75003 Paris (FR)
(72) Inventeur: AGNUS, Benoit, F-94360 Bry sur Marne (FR); BESINS, Antoine, F-75017 Paris (FR)
(74) Mandataire: Nargolwalla, Cyra
(86) Numéro de dépôt international: PCT/FR1999/000515
(87) Numéro de publication internationale: WO 1999/045932

(56) Documents cités:
- EP-A- 0 371 466
- WO-A-95/05807
- WO-A-98/48782
- FR-A- 2 399 243
- US-A- 4 755 386
- US-A- 5 633 242

## Description

La présente invention a pour objet une composition pharmaceutique à base de progestérone naturelle de synthèse et d'oestradiol se présentant sous la forme d'un comprimé, ainsi qu'un procédé pour sa préparation.

Dans le contexte de la présente invention, on entend par "progestérone naturelle de synthèse" une progestérone synthétisée dont la formule chimique correspond à la progestérone "naturelle", telle qu'on la trouve dans l'organisme féminin. En revanche, les "progestatifs de synthèse" sont des molécules entièrement synthétiques telles la trimégestone, la noréthistérone et autres, dont la structure ne correspond pas à celle de la progestérone naturelle.

L'état de la ménopause chez la femme peut entraîner, voire aggraver, certaines pathologies telles que l'ostéoporose ou les accidents cardio-vasculaires.

Les oestrogènes, en particulier l'oestradiol, sont alors prescrits afin de réduire ces conséquences néfastes de la ménopause. Mais l'administration des oestrogènes peut en elle-même provoquer d'autres effets indésirables.

Il est donc habituel d'y associer un traitement de progestérone afin d'éviter notamment les risques d'hyperplasie de l'endomètre.

Il est évidemment avantageux de pouvoir proposer à la femme ces deux principes actifs associés dans le même médicament afin de permettre l'absorption par une prise unique.

On rencontre déjà sur le marché des médicaments à base de progestérone et d'oestradiol qui se présentent sous la forme de comprimés. Cependant, tous les comprimés connus à cette date utilisent des progestatifs de synthèse qui n'ont pas tous les effets thérapeutiques de la progestérone naturelle de synthèse et peuvent même avoir des effets indésirables. L'utilisation de la progestérone naturelle de synthèse permet d'éviter les effets notamment hépatotoxiques des progestatifs de synthèse.

En ce qui concerne la progestérone naturelle, la Société Demanderesse a déjà mis au point un médicament qui connaît un très grand succès sur le plan thérapeutique. Ce médicament se présente sous la forme d'une capsule contenant la progestérone naturelle de synthèse micronisée dans une suspension d'huile. Ce médicament est vendu sous la marque UTROGESTAN.

Au vu des très bons résultats que ce médicament procure quand il est associé avec un comprimé conventionnel d'oestradiol dans le traitement des désagréments associés à la ménopause, il serait très intéressant de pouvoir disposer d'un comprimé à base de progestérone naturelle et d'oestradiol, ayant une bioéquivalence par rapport aux capsules d'UTROGESTAN administrées en association avec un comprimé d'oestradiol.

Mais il se trouve que la mise au point d'un tel comprimé entraîne un certain nombre de problèmes.

Ces problèmes proviennent essentiellement du fait que la progestérone naturelle, pour être efficace, doit être employée à des doses beaucoup plus fortes que les progestatifs de synthèse, à savoir 50 ou 60 fois plus de principe actif par rapport au comprimé contenant des progestatifs de synthèse. Ceci a pour conséquence que le rapport entre progestérone naturelle et oestradiol est de l'ordre de 100/1, provoquant des problèmes au niveau de l'homogénéité du mélange des principes actifs, aussi bien dans les comprimés que pendant leur fabrication.

Une autre conséquence se situe au niveau du pourcentage d'excipients pouvant être inclus dans les comprimés. En effet, du fait de la quantité importante en progestérone naturelle, il est nécessaire de diminuer considérablement la teneur en excipients afin de respecter les contraintes de taille et de poids propres à des comprimés destinés à une administration par voie orale et/ou vaginale.

Les excipients dans les comprimés, on le sait, jouent des rôles divers. Ils servent à augmenter la stabilité des principes actifs, à permettre l'obtention d'un profil de libération particulier selon leur nature:, mais ils servent surtout à faciliter la compression des différents ingrédients afin d'obtenir un comprimé possédant de bonnes caractéristiques de dureté, de désagrégation, et de dissolution.

Or, après de longs et nombreux travaux et recherches, la société Demanderesse est parvenue à mettre au point un nouveau comprimé à base de progestérone naturelle et d'oestradiol, répondant aux exigences d'homogénéité du mélange et ne contenant que de faibles quantités d'excipients permettant ainsi l'obtention de comprimés d'une taille et d'un poids tout à fait acceptables pour une administration par voie orale et/ou vaginale.

De plus, la Société Demanderesse a pu constater que lorsque ces comprimés présentaient un profil de désagrégation particulier, ils présentaient également une bioéquivalence par rapport aux capsules d'UTROGESTAN en association avec un comprimé d'oestradiol, ce qui a pu être démontré par une étude pharmacocinétique comparant les deux traitements. Ce profil est caractérisé par un temps de désagrégation inférieur à 15 minutes, de préférence inférieur à 10 minutes, et plus préférentiellement encore inférieur à 5 minutes.

Le temps de désagrégation des comprimés est mesuré dans un test "D". Selon ce test "D", un comprimé selon l'invention est placé dans un bécher d'un litre contenant 700 ml d'eau distillée portés à une température de 37°C, et subit un mouvement alternatif vertical. Le temps nécessaire pour une perte de cohésion totale est mesuré.

Le document WO 9 505 807 décrit des comprimés de progestérone et d'oestradiol contenant du polyéthylène glycol et ayant un temps de désagrégation supérieur à 14 minutes.

L'invention concerne donc une composition pharmaceutique à base de progestérone naturelle et d'oestradiol se présentant sous forme de comprimé, caractérisée par le fait qu'elle présente un temps de désagrégation inférieur à 10 minutes, et plus préférentiellement encore inférieur à 5 minutes.

Le rapport en poids entre l'oestradiol et la progestérone est compris entre 5/100 et 1/100, ce rapport étant exprimé en fonction du poids total de la progestérone dans le comprimé.

L'homogénéité du mélange et le ratio oestradiol/progestérone ont pu être obtenus grâce à un procédé de granulation humide. L'oestradiol est incorporé dans une solution liante avant d'être soumis à une étape de mouillage-granulation, garantissant ainsi une bonne homogenéité du mélange ainsi qu'un bon dosage de ce steroïde.

Les comprimés mis au point par la Société Demanderesse, bien que contenant des quantités d'excipients significativement inférieures à celles des comprimés de l'art antérieur, présentent néanmoins d'excellentes caractéristiques de stabilité, de dureté, de désagrégation et de dissolution.

Selon un mode préférentiel de réalisation, la composition pharmaceutique sous forme de comprimé selon l'invention est caractérisée par le fait que sa teneur en excipients est d'au plus 20%, de préférence d'au plus 17%, et plus préférentiellement encore d'au plus 15 %, les pourcentages étant exprimés en poids par rapport à la matière sèche totale du comprimé.

Comme excipients susceptibles d'être utilisés dans la composition pharmaceutique selon l'invention, on peut citer les agents diluants, les agents désintégrants, les agents lubrifiants, les agents liants et les agents de pelliculage.

Comme exemples d'agents diluants, on peut citer les amidons, les polyols et les celluloses. De préférence, le comprimé selon l'invention contient notamment de la carboxyméthylcellulose sodique.

Comme exemples d'agents désintégrants on peut citer les carboxyméthylcelluloses, l'acide alginique ainsi que son sel de sodium, et les amidons. De préférence, le comprimé conforme à l'invention contient de la carboxyméthylcellulose sodique réticulée non seulement à titre de diluant mais également grâce à ses qualités d'agent désintégrant, ainsi que pour son fort pouvoir absorbant.

L'agent lubrifiant préféré dans le cadre de la présente invention est le stéarate de magnésium.

Parmi les agents liants préférés dans le cadre de la présente invention, on peut citer les polyvinylpyrollidones, mais également la carboxyméthylcellulose sodique. Ce produit permet non seulement l'obtention d'une suspension stable d'oestradiol, mais également une libération quasi-immédiate du principe actif. De plus il est d'une mise en oeuvre très aisée.

Les agents de pelliculage préférés dans le cadre de la présente invention sont la méthylhydroxypropylcellulose et le polyethylèneglycol 600. Il est en effet nécessaire de procéder au pelliculage afin d'éviter une contamination pure.

Un avantage du comprimé selon la présente invention est qu'il présente un profil de dissolution tel que la teneur en progestérone libérée este d'au moins de 75 % et que la teneur en oestradiol libéré est d'au moins de 75% en 15 minutes, de préférence en 10 minutes, et plus préférentiellement encore en 5 minutes.

Le comprimé selon l'invention présente une dureté comprise entre 10 et 80N, de préférence comprise entre 20 et 70 N, et plus préférentiellement encore comprise entre 30 et 60 N.

L'invention concerne également un procédé pour la préparation d'une composition pharmaceutique à base de progestérone et d'oestradiol se présentant sous forme d'un comprimé. Ce procédé est caractérisé par le fait que :
- on prépare une suspension de mouillage que l'on mélange avec une suspension d'oestradiol,
- on prépare un premier mélange de progestérone et de diluant,
- on prépare un deuxième mélange du premier mélange et de la suspension d'oestradiol mouillée,
- on procède à un deuxième mouillage du produit de ce deuxième mélange,
- on procède à une granulation du produit du mouillage, afin d'obtenir un produit granulaire,
- on ajoute des agents désintégrants et diluants au produit granulaire afin d'obtenir un troisième mélange,
- on ajoute un lubrifiant au troisième mélange afin d'obtenir un quatrième mélange,
- on effectue une compression de ce quatrième mélange afin d'obtenir des comprimés,
- on procède au pelliculage des comprimés.

Selon un mode préférentiel de réalisation de l'invention, la granulation est suivie par un démottage, un séchage, puis un calibrage.

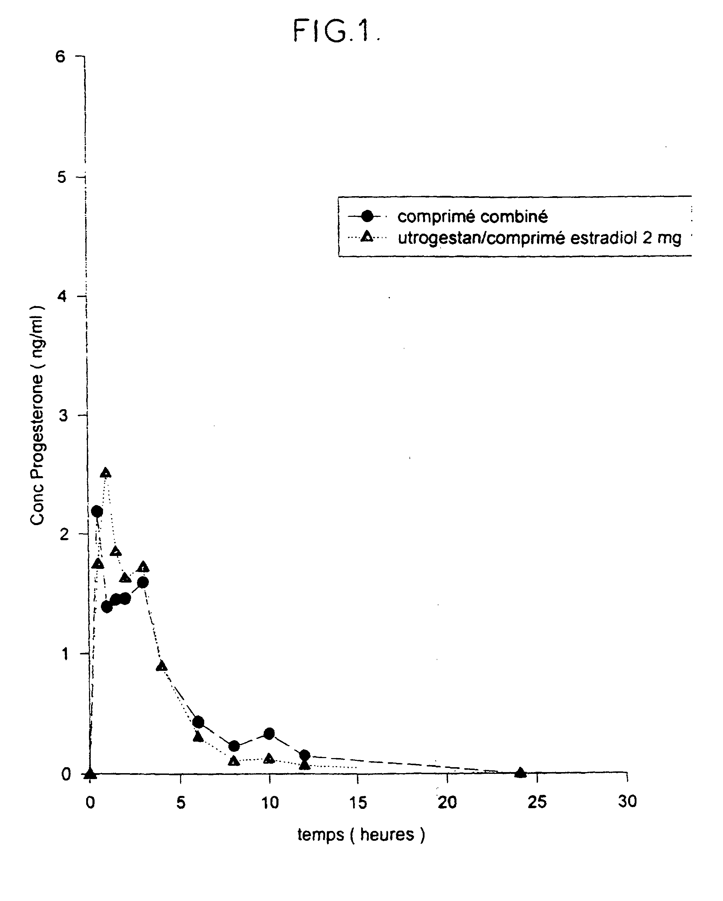

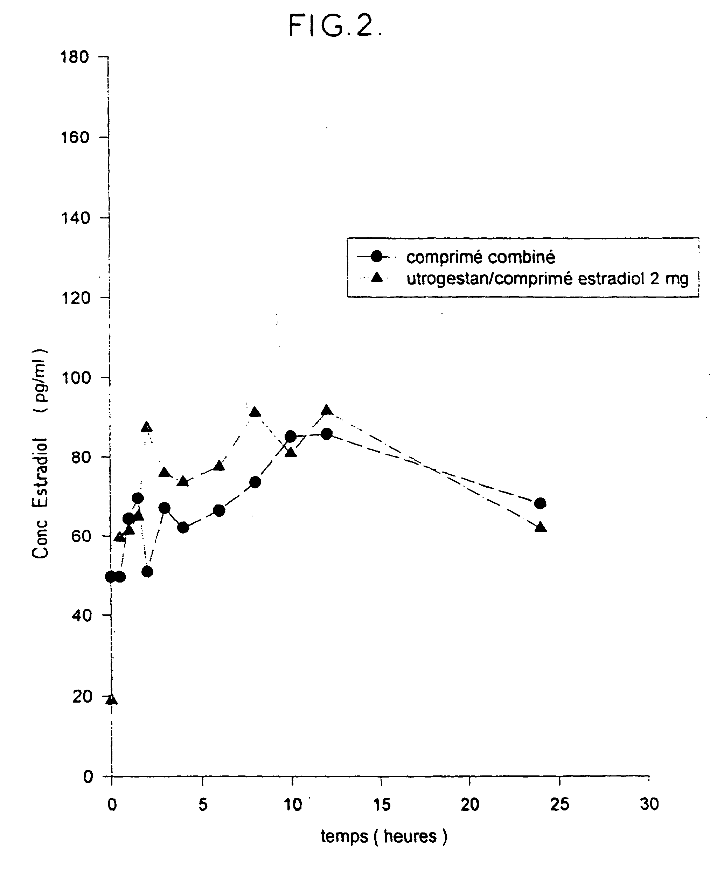

Les Figures 1 et 2 montrent les résultats d'une étude pharmacocinétique effectuée sur six femmes caucasiennes ménopausées, âgées de moins de 70 ans, qui compare les comprimés de la présente invention aux capsules d'UTROGESTAN administrées en association avec un comprimé d'oestradiol.

Ces Figures confirment la bonne corrélation entre un comprimé associant de la progestérone et de l'estradiol, versus une capsule d'utrogestan et un comprimé d'estradiol.

L'invention sera mieux comprise à l'aide des exemples non limitatifs décrits ci-dessous.

### EXEMPLE 1 : COMPOSITION PHARMACEUTIQUE SELON L'INVENTION

Les compositions de comprimés à base de progestérone naturelle et d'oestradiol selon l'invention contenant 101 mg de principes actifs sont données au Tableau I ci-dessous.

**TABLEAU 1**

| **NOM DU COMPOSANT** | **FONCTION** | **QUANTITE mg/comprimé** |
|---|---|---|
| Progestérone micronisée | Progestatif | 100 |
| Oestradiol micronisé | Oestrogène | 1 |
| Carboxyméthyl cellulose sodique | Liant | 3,20 |
| Carboxyméthyl cellulose sodique réticulée | Diluant désintégrant | 10,75 |
| Stéarate de magnésium | Lubrifiant | 1,16 |
| Méthylhydroxypropyl cellulose | Agent de pelliculage | 1,80 |
| Polyéthylène glycol 600 | Agent de pelliculage | 0,20 |

### EXEMPLE 2 : PREPARATION DE COMPRIMES SELON L'INVENTION

Un lot de comprimés contenant 100 mg de progestérone naturelle et 1 mg d'oestradiol par comprimé a été préparé comme décrit ci-dessous.

### a) Préparation du mélange de progestérone

On mélange 10 kg de progestérone micronisée (obtenues auprès de la Société DIOSYNTH, à Oss, Pays-Bas) et 500 g de carboxyméthylcellulose sodique réticulée dans la cuve d'un mélangeur granulateur planétaire du type BONNET équipé d'un axe d'agitation du type Lyre, pendant 10 minutes, afin d'obtenir un mélange homogène.

### b) Préparation de la suspension de mouillage d'oestradiol

On introduit 6 litres d'eau purifiée dans un récipient en acier inoxydable. On agite à l'aide d'un mélangeur-malaxeur TURBOTEST RAYNERI équipé d'un axe d'agitation de type défloculeuse.

On y verse progressivement 320 g de carboxyméthyle cellulose sodique (commercialisée sous la marque BLANOSE 7LF par la Société HERCULES), et puis on homogénéise jusqu'à la dissolution complète de la BLANOSE. A cette solution de BLANOSE, on ajoute 102 g d'estradiol. On ajoute pendant 45 minutes jusqu'à dispersion complète de l'estradiol.

### c) Mouillage

On verse la solution de mouillage d'estradiol en filet sur le mélange de poudre contenu dans le mélangeur BONNET, l'axe de type Lyre étant programmé sur la vitesse 1 pendant 5 minutes.

### d) Granulation

On homogénéise le mélange pendant 30 minutes après la fin du mouillage afin d'obtenir une poudre granulaire.

### e) Séchage

Le séchage est pratiqué dans une étuve à 40°C pendant 6 heures.

### f) Calibrage

Le calibrage de la poudre granulaire est réalisé à l'aide d'un granulateur oscillant FREWITT équipé d'une grille d'inox de diamètre de maille de 1250 µm.

### g) Ajout des agents désintégrants et diluants au produit granulaire

A 10 kg du produit granulaire, on ajoute 550 g de carboxyméthyl cellulose sodique réticulée dans la cuve d'un mélangeur ROUE RÖHN et on mélange pendant deux fois cinq minutes.

Au mélange ainsi obtenu, on ajoute 112 g de stéarate de magnésium et on mélange pendant trois minutes afin d'obtenir le mélange final.

### h) Compression du mélange final

La compression du mélange final est effectuée sur une machine à comprimer rotative de type FROGERAIS MR 200, équipée de poinçons. Le réglage de la machine est effectué de manière à obtenir pour des comprimés de masse unitaire de 116 mg et un temps de désagrégation inférieur à environ 5 minutes et une dureté d'environ 40 N.

### i) Pelliculage

On prépare la solution de pelliculage en ajoutant 270 g de méthylhydroxypropylcellulose (commercialisé sous le nom de PHARMACOAT 606 par la Société SEPPIC) et 30 g de polyéthylène glycol 600 à 3,2 litres d'eau purifiée. On mélange pendant 1 heure puis on pulvérise les comprimés avec un pistolet AirLess type STELLERLUPT et d'une buse de diamètre 0,5 mm.

Les comprimés obtenus sont conditionnés en plaquettes alvéolées, thermoformées, constituées d'une feuille de PVC d'une épaisseur de 250 µm, scellée à une feuille d'aluminium d'une épaisseur de 20 µm.

Une étude de stabilité a été réalisée sur les comprimés ainsi préparés.

Aucune modification des paramètres galéniques et analytiques n'a été observée à la fin de cette période, démontrant ainsi la bonne stabilité des comprimés selon l'invention.

### EXEMPLE 3 : TEMPS DE DESAGREGATION DES COMPRIMES SELON L'INVENTION

Un comprimé fabriqué selon la méthode décrite dans l'exemple 2 a été placé dans un bécher contenant 700 ml d'eau distillée, portés à une température de 37°C. Le temps de désagrégation correspondant à une perte de cohésion totale était de 4 minutes.

### EXEMPLE 4 : TEMPS DE DISSOLUTION DES COMPRIMES SELON L'IN-VENTION

On a pris 6 cuves de dissolution de capacité 1000ml.

### Pour la progestérone

Dans chacune des cuves, on a placé un comprimé dans un milieu de dissolution contenant 1000 ml d'une solution aqueuse contenant 1% (m/v) de sodium laurylsulfate. On a ensuite agité la solution dans chaque cuve à l'aide d'un appareil de dissolution à pales tournantes PHARMATEST type PTWS III. Les pales ont été immergées dans le milieu de dissolution à une distance de 25 mm ± 2 mm entre la pale et le fond de la cuve. Les pales ont été mises sous une agitation de 75 tours par minute.

1 ml du milieu de dissolution a été prélevé toutes les 5 minutes dans chacune des cuves.

Chaque échantillon a été dosé par HPLC (= 280 ηm) après injection de 20 µl de solution à analyser.

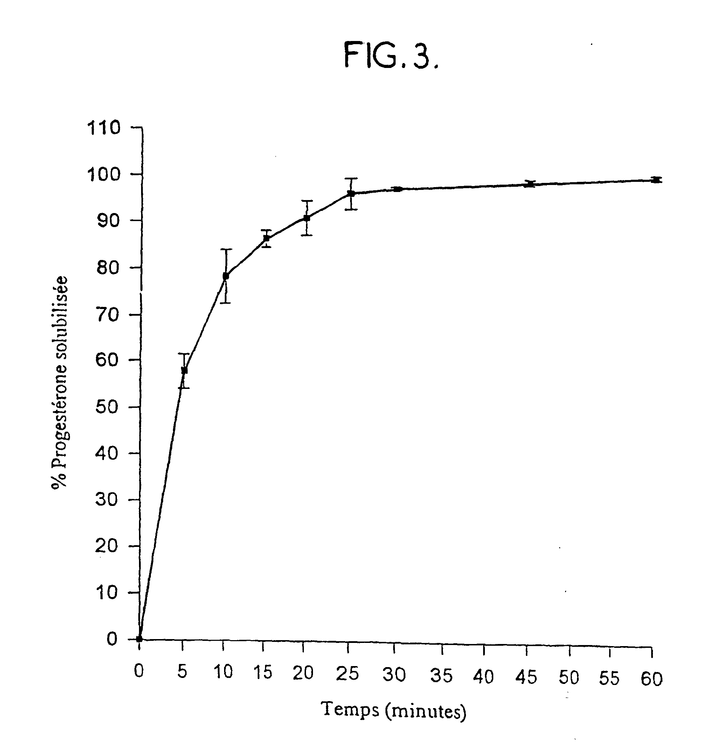

La courbe donnée dans la Figure 3 montre le profil de dissolution obtenu. Au moins 75% de la progestérone est libérée à 15 minutes de dissolution.

### Pour l'estradiol

Dans chacune des cuves on a placé 2 comprimés dans un milieu de dissolution contenant 500 ml d'une solution aqueuse contenant 0,5% (m/v) de sodium laurylsulfate. On a ensuite agité la solution dans chaque cuve à l'aide d'un appareil de dissolution à pales tournantes PHARMATEST type PTWS III. Les pales ont été immergées dans le milieu de dissolution à une distance de 25 mm ± 2 mm entre la pale et le fond de la cuve. Les pales ont été mises sous une agitation de 75 tours par minute.

1 ml du milieu de dissolution a été prélevé toutes les 5 minutes dans chacune des cuves.

Chaque échantillon a été dosé par HPLC (= 280 ηm) après injection de 20 µl de solution à analyser.

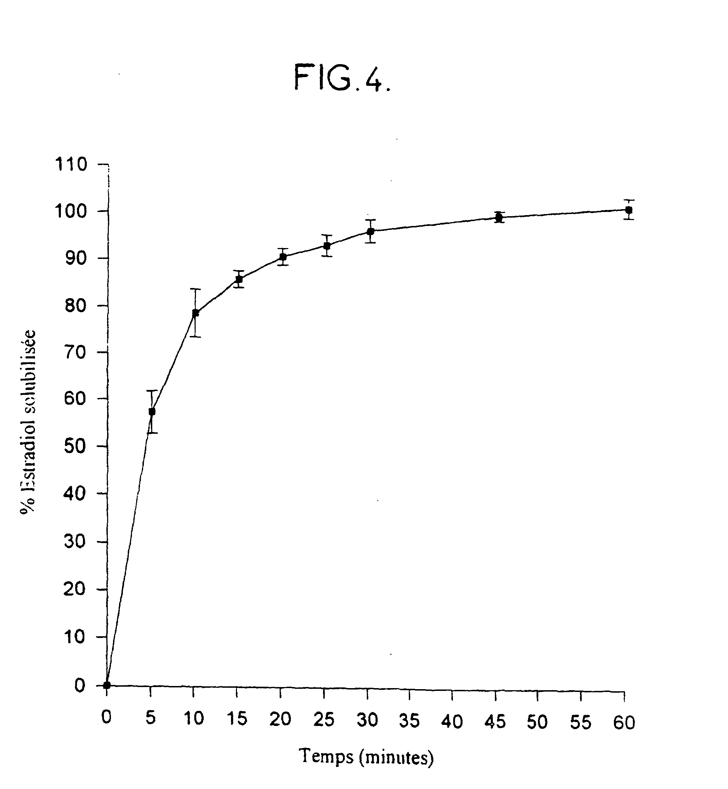

La courbe donnée dans la Figure 4 démontre le profil de dissolution obtenu. Au moins 75% de l'estradiol est libérée à 15 minutes de dissolution.

## Revendications

1. Composition pharmaceutique à base d'oestradiol et de progestérone naturelle de synthèse dans un rapport compris entre 5/100 et 1/100 en poids, se présentant sous la forme de comprimé, susceptible d'être obtenue par un procédé comprenant les étapes suivantes :
- on prépare une suspension de mouillage que l'on mélange avec une suspension d'oestradiol,
- on prépare un premier mélange de progestérone et de diluant,
- on prépare un deuxième mélange de ce premier mélange et de la suspension d'oestradiol mouillée,
- on procède à un deuxième mouillage du produit de ce mélange,
- on procède à une granulation du produit du mouillage, afin d'obtenir un produit granulaire,
- on ajoute des agents désintégrants et diluants au produit granulaire afin d'obtenir un troisième mélange,
- on ajoute un lubrifiant au troisième mélange afin d'obtenir un quatrième mélange,
- on effectue une compression de ce quatrième mélange afin d'obtenir des comprimés,
- on procède au pelliculage des comprimés,
ladite composition présentant un temps de désagrégation inférieur à 10 minutes et plus préférentiellement inférieur à 5 minutes.

2. Composition pharmaceutique selon la revendication 1, **caractérisée par le fait que** sa teneur en excipients est d'au plus 20%, de préférence d'au plus 17%, et plus préférentiellement encore d'au plus 15 %, les pourcentages étant exprimés en poids par rapport à la matière sèche totale du comprimé.

3. Composition pharmaceutique selon l'une quelconque des revendications 1 ou 2, **caractérisée par le fait qu'**elle présente un profil de dissolution tel que la teneur en progestérone libérée est d'au moins 75 % et que la teneur en oestradiol libéré est d'au moins 75% en 15 minutes, de préférence en 10 minutes, et plus préférentiellement encore en 5 minutes.

4. Composition pharmaceutique selon l'une quelconque des revendications 1 à 3, **caractérisée par le fait qu'**elle présente une dureté comprise entre 10 et 80N, de préférence comprise entre 20 et 70N, et plus préférentiellement encore comprise entre 30 et 60N.

5. Composition pharmaceutique selon l'une quelconque des revendications 1 à 4, **caractérisée par le fait qu'**elle contient des agents diluants, des agents désintégrants, des agents lubrifiants, des agents liants, et des agents de pelliculage.

6. Procédé de préparation d'une composition pharmaceutique selon l'une quelconque des revendications 1 à 5, **caractérisée par le fait que** :
- on prépare une suspension de mouillage que l'on mélange avec une suspension d'oestradiol,
- on prépare un premier mélange de progestérone et de diluant,
- on prépare un deuxième mélange de ce premier mélange et de la suspension d'oestradiol mouillée,
- on procède à un deuxième mouillage du produit de ce mélange,
- on procède à une granulation du produit du mouillage, afin d'obtenir un produit granulaire,
- on ajoute des agents désintégrants et diluants au produit granulaire afin d'obtenir un troisième mélange,
- on ajoute un lubrifiant au troisième mélange afin d'obtenir un quatrième mélange,
- on effectue une compression de ce quatrième mélange afin d'obtenir des comprimés,
- on procède au pelliculage des comprimés.

## Patentansprüche

1. Pharmazeutische Zusammensetzung auf Basis von Östradiol und synthetischem natürlichem Progesteron in einem Gewichtsverhältnis zwischen 5/100 und 1/100 in Form einer Tablette, die nach einem Verfahren erhältlich ist, bei dem man:
- eine Benetzungssuspension herstellt, die man mit einer Östradiolsuspension vermischt,
- eine erste Mischung von Progesteron und Verdünnungsmittel herstellt,
- eine zweite Mischung aus der ersten Mischung und der benetzten Östradiolsuspension herstellt,
- eine zweite Benetzung des dabei erhaltenen Produkts durchführt,
- eine Granulierung des Benetzungsprodukts durchführt, zu erhalten ein Granulatprodukt,
- dem Granulatprodukt Auflösungsmittel und Verdünnungsmittel zusetzt, um eine dritte Mischung zu erhalten,
- der dritten Mischung ein Gleitmittel zusetzt, um eine vierte Mischung zu erhalten,
- diese vierte Mischung zu Tabletten verpreßt und
- eine Filmbeschichtung der Tabletten durchführt,
wobei die Zusammensetzung eine Zerfallszeit von weniger als 10 Minuten und weiter bevorzugt weniger als 5 Minuten aufweist.

2. Pharmazeutische Zusammensetzung nach Anspruch 1, **dadurch gekennzeichnet, daß** sie einen Hilfsstoffgehalt von höchstens 20 Gew.-%, vorzugsweise höchstens 17 Gew.-% und weiter bevorzugt höchstens 15 Gew.-%, bezogen auf die gesamte Trockensubstanz der Tablette, aufweist.

3. Pharmazeutische Zusammensetzung nach Anspruch 1 oder 2, **dadurch gekennzeichnet, daß** sie ein solches Auflösungsprofil aufweist, daß in 15 Minuten, vorzugsweise in 10 Minuten und noch weiter bevorzugt in 5 Minuten der Gehalt an freigesetztem Progesteron mindestens 75% und der Gehalt an freigesetztem Östradiol mindestens 75% beträgt.

4. Pharmazeutische Zusammensetzung nach einem der Ansprüche 1 bis 3, **dadurch gekennzeichnet, daß** sie eine Härte zwischen 10 und 80 N, vorzugsweise zwischen 20 und 70 N und noch weiter bevorzugt zwischen 30 und 60 N aufweist.

5. Pharmazeutische Zusammensetzung nach einem der Ansprüche 1 bis 4, **dadurch gekennzeichnet, daß** sie Verdünnungsmittel, Auflösungsmittel, Gleitmittel, Bindemittel und Filmbeschichtungsmittel enthält.

6. Verfahren zur Herstellung einer pharmazeutischen Zusammensetzung nach einem der Ansprüche 1 bis 5, **dadurch gekennzeichnet, daß** man:
- eine Benetzungssuspension herstellt, die man mit einer Östradiolsuspension vermischt,
- eine erste Mischung von Progesteron und Verdünnungsmittel herstellt,
- eine zweite Mischung aus der ersten Mischung und der benetzten Östradiolsuspension herstellt,
- eine zweite Benetzung des dabei erhaltenen Produkts durchführt,
- eine Granulierung des Benetzungsprodukts durchführt, um ein Granulatprodukt zu erhalten,
- dem Granulatprodukt Auflösungsmittel und Verdünnungsmittel zusetzt, um eine dritte Mischung zu erhalten,
- der dritten Mischung ein Gleitmittel zusetzt, um man eine vierte Mischung zu erhalten,
- diese vierte Mischung zu Tabletten verpreßt und
- eine Filmbeschichtung der Tabletten durchführt.

## Claims

1. A pharmaceutical composition with an oestradiol and synthetic natural progesterone base in a weight ratio of between 5/100 and 1/100, in the form of a tablet, obtainable by a process comprising the following steps :
- a wetting agent suspension is prepared which is mixed with an oestradiol suspension,
- a first progesterone and diluent mixture is prepared,
- a second mixture of this first mixture and of the wetted oestradiol suspension is prepared,
- a second wetting of the product of this mixture is carried out,
- a granulation of the product of the wetting is carried out in order to obtain a granular product,
- disintegrating agents and diluents are added to the granular product in order to obtain a third mixture,
- a lubricant is added to the third mixture in order to obtain a fourth mixture,
- a compression of this fourth mixture is carried out in order to obtain tablets,
- stripping of the tablets is carried out,
said composition having a disintegration time of less than 10 minutes and more preferably of less than 5 minutes.

2. The pharmaceutical composition according to claim 1, **characterised in that** its excipient content is at most 20%, preferably at most 17%, and more preferably still at most 15 %, the percentages being expressed by weight relative to the total dry matter of the tablet.

3. The pharmaceutical composition according to any of claims 1 or 2, **characterised in that** it has a dissolution profile such that the released progesterone content is at least 75 % and that the released oestradiol content is at least 75% in 15 minutes, preferably in 10 minutes, and more preferentially still in 5 minutes.

4. The pharmaceutical composition according to any of claims 1 to 3, **characterised in that** it has a hardness between 10 et 80 N, preferably between 20 and 70 N, and more preferentially still between 30 and 60 N.

5. The pharmaceutical composition according to any of claims 1 to 4, **characterised in that** it contains diluents, disintegrating agents, lubricants, binding agents and stripping agents.

6. The pharmaceutical composition according to any of claims 1 to 5, **characterised in that**:
- a wetting agent suspension is prepared which is mixed with an oestradiol suspension,
- a first progesterone and diluent mixture is prepared,
- a second mixture of this first mixture and of the wetted oestradiol suspension is prepared,
- a second wetting of the product of this mixture is carried out,
- a granulation of the product of the wetting is carried out in order to obtain a granular product,
- disintegrating agents and diluents are added to the granular product in order to obtain a third mixture,
- a lubricant is added to the third mixture in order to obtain a fourth mixture,
- a compression of this fourth mixture is carried out in order to obtain tablets,
- stripping of the tablets is carried out.
